# EUROPEAN PATENT APPLICATION

(11) **EP 3 764 366 A1**
(43) Date of publication of application: **13.01.2021**
(21) Application number: 19185127.8
(22) Date of filing: 09.07.2019
(51) Int. Cl.: G16H 30/40, G16H 50/70, G16H 50/50

(54) **UNCERTAINTY MAPS FOR DEEP LEARNING ELECTRICAL PROPERTIES TOMOGRAPHY**

(71) Applicant: Koninklijke Philips N.V., 5656 AG Eindhoven (NL)
(72) Inventor: KATSCHER, Ulrich, 5656 AE Eindhoven (NL); HAMPE, Nils, 5656 AE Eindhoven (NL)
(74) Representative: de Haan, Poul Erik

(57) **Abstract**

The present disclosure relates to a method for determining electrical properties, EP, of a target volume (708) in an imaged subject (718). The method comprises: a) training (201) a deep neural network, DNN, using a training dataset, the training dataset comprising training B1 field maps and corresponding EP maps, the training comprising using a monte carlo, MC, dropout of the DNN during the training, resulting in a trained DNN configured for generating EP maps from B1 field maps; b) receiving (203) an input B1 field map of the target volume, and repeatedly generate by the trained DNN from the input B1 field map an EP map, resulting in a set of EP maps, wherein the generating comprises using in each repetition the MC dropout during inference of the DNN; c) combining (205) the set of EP maps for determining an EP map and associated uncertainty map of the input B1 field map.

## Description

### FIELD OF THE INVENTION

The invention relates to scanning imaging systems, in particular to a medical analysis system for determining electrical properties (EP) of a target volume in an imaged subject.

### BACKGROUND OF THE INVENTION

Electric Properties Tomography (EPT) is a technique to measure electric properties of tissue non-invasively and quantitatively in-vivo by post-processing of the magnitude and phase of B1 field distributions. The relation between EPs and complex B 1 values is given by the so-called Helmholtz-equation. Thus, taking the complex valued B1 map from MRI as input, the Helmholtz equation provides the EPs. However, conventional EPTs suffer from the transceive phase problem, issues at tissue boundaries and noise amplification.

### SUMMARY OF THE INVENTION

Various embodiments provide for medical analysis system for determining electrical properties (EP) of a target volume in an imaged subject, method, and computer program product, as described by the subject matter of the independent claims. Advantageous embodiments are described in the dependent claims.

Embodiments of the invention may provide for a means for utilization of deep learning for EPT in order to solve essential problems of the conventional methods for EPT such as the transceive phase problem, issues at tissue boundaries and noise amplification. This may be achieved by using the Monte Carlo dropout in deep neural networks, to produce uncertainty values along with network's EP output. The uncertainty values serve as an indicator for faulty network behavior of the deep neural networks.

In one aspect, the invention relates to a medical analysis system for determining electrical properties, EP, of a target volume in a subject. The subject may for example be imaged or scanned by an MRI system, so as to generate images of the target volume in the subject. The medical analysis system comprises at least one processor; and at least one memory storing machine executable instructions, the processor being configured for controlling the medical analysis system, wherein execution of the machine executable instructions causes the processor to: a) train of a deep neural network, DNN, using a training dataset, the training dataset comprising training B1 field maps and corresponding EP maps, the training comprising using a monte carlo, MC, dropout of the DNN during the training, resulting in a trained DNN configured for generating EP maps from B1 field maps; b) receive an input B1 field map of the target volume, and repeatedly generate by the trained DNN from the input B1 field map an EP map, resulting in a set of EP maps, wherein the generating comprises using in each repetition the MC dropout during inference (or usage) of the trained DNN; c) combine the set of EP maps for determining an EP map and associated uncertainty map of the input B1 field map.

The training set may be built using existing B1 field maps of a volume. The B1 field maps are labeled with respective EP maps. That volume may be the target volume or another subject's volume. For example, numerous B1 mapping methods exist for experimentally determining B1 field distributions. These methods may be used for measuring magnitude and phase distributions. For example, two MRI scans may be used to acquire or generate a complex-valued B1 field map e.g. one scan for acquiring the B1 magnitude distribution and one scan for acquiring the B1 phase distribution. The B1 field maps maybe B1 transmit field maps. For example, magnetic resonance electrical property tomography (MR-EPT) maybe used for estimating the conductivity and permittivity distribution of the target volume. Imaging in accordance with MR-EPT may enable to obtain a phase image and/or B1 amplitude image of the RF field produced using specific pulse sequences. For example, the subject may be imaged by a magnetic resonance imaging (MRI) system, resulting in MRI data. The MRI data may be used to reconstruct tissue EPs by solving an electromagnetic inverse problem relating MR measurements of the transmit radio frequency (RF) field to the EPs.

When the information on B1 field is determined or presented in the form of a 2-D or 3-D spatial distribution, it may be called a B1 field map. The B1 field map may for example be determined using MRI data acquired by scanning the target volume of the subject. A B1 field map may be used to derive from the B1 field map an EP map. The derived EP map therefore corresponds to that B1 field map. Deriving the EP map comprises performing an EPT reconstruction using the B1 field map e.g. the EP map may be computed by taking Laplacian of the B1 field map.

The present subject matter uses the MC dropout for inferring an uncertainty value along with each EP value voxel by voxel. The uncertainty values serve as an indicator for faulty network behavior of the DNN with the possibility of potentially reducing in-vivo artifacts by repeating the scan.

In patch-based reconstructions, utilization of overlapping patches can yield different EP values for each voxel. Availability of uncertainties for each EP value allows for preferring values with low uncertainty, therefore yielding higher reconstruction accuracy. In particular, the uncertainty could be used to determine weighting factors for EP values from overlapping patches, preferring low uncertainty EPs and therefore increasing reconstruction accuracy. The patch based reconstruction may for example be performed by inputting to the trained DNN a patch of pixels or voxels extracted from a full image of a B1 map in order to reconstruct another patch of EP values using the trained DNN. For example, multiple patches of the same B1 map may be input to the trained DNN in order to reconstruct respective multiple patches of EP values. As the multiple input patches may be overlapping patches as they may share pixels of the B1 map, the multiple patches of EP values (EP patches) may be overlapping as well in that a same pixel may have a value in one EP patch and another value in another EP patch. Using the MC dropout in the patch based reconstruction; each of the multiple EP patches may be associated with respective uncertainties. This may for example result in a same pixel having different pairs of (EP value, uncertainty). This may allow a more sophisticated weighting of overlapping values in patches by taking values with lower uncertainty into account.

Using the DNN may be advantageous as it may enable to model linear as well as complex non-linear relationships. This may particularly be advantageous in case the amount of subject data is large. In addition, dropout layers are utilized to introduce disturbances in the DNN by randomly deactivating connections between neurons of the DNN. With the present subject matter, the MC dropout, in particular the dropout rate (i.e. the fraction of weights set to zero) remains non-zero (common value is 0.5) when applying the trained DNN to test data after the training. Doing so, a non-deterministic network behavior of the DNN is obtained, causing differences in network outputs for repeated application to the same input with differences increasing with increasing EP uncertainties.

The present subject matter may be applied for cases where electric properties are of diagnostic value, or where electric properties are needed for RF safety management, or where electric properties are needed for therapy planning like RF ablation / hyperthermia.

According to one embodiment, the processor is further caused to determine if the uncertainty map fulfils a predefined quality condition, and in response to determining that the uncertainty map does not fulfil the predefined quality condition, re-train the DNN using a further training dataset, and repeat steps b)-c) using the retrained DNN instead of the trained DNN.

In one example, determining that the uncertainty map does not fulfil the predefined quality condition may automatically be performed or may be performed by prompting a user for requesting if the predefined quality condition is fulfilled. In particular, providing uncertainty maps along with the EP maps offer the chance for clinicians to rate EP-based findings. For example, thresholding of uncertainty values (e.g. the quality condition may require a maximum uncertainty value for a predefined portion of (x%) of values in a map) could be used as an emergency break to identify faulty network behavior, thereby increasing overall trustworthiness of deep learning based EPT. In case in-vivo artifacts are the cause of high uncertainty, scan repetition may yield an increased result quality.

According to one embodiment, the further training dataset is larger than the training dataset.

The further training dataset comprises the training dataset in addition to further pairs of B1 field maps and corresponding EP maps. Using a larger dataset maybe advantageous as it may increase the variability of the training dataset. The variability refers to variability of anatomical geometry and tissue conductivity values. Experiments show that neural networks are able to approximate the desired underlying functionality to great accuracy for prediction data that is similar to the training data. A large variability within the training set has a beneficial effect of increasing the likelihood of similarity between training data and prediction data used during the inference.

According to one embodiment, the processor is further caused to: perform the repeating until the uncertainty map fulfils the predefined quality condition. The training dataset of each current iteration may be obtained by increasing the training dataset of a last iteration by adding pairs of B1 field maps and corresponding EP maps. After the predefined quality condition is fulfilled, the trained DNN may be used multiple times (i.e. steps b) and c) may be repeated) for input B1 field maps of prediction data.

According to one embodiment, the training B1 field maps comprise B1 field phase maps and/or B1 amplitude maps, wherein the input B1 field map comprises a B1 phase map and/or B1 amplitude map.

In a first example, each of the training B1 field maps comprises a B1 field phase map, wherein the input B1 field map comprises a B1 phase map. In other terms, the training is performed using B1 phase maps in order to generate a trained DNN that can estimate or predict a conductivity map in response to receiving as input a B 1 phase map. The DNN may for example comprise an input layer configured to receive voxel or pixel values of the training B1 maps. Each training B1 map may for example comprise an image whose pixel values may be input to the DNN for performing the training. The output layer of the DNN may for example be configured to provide output values which are indicative of the conductivity values. The training may result in the DNN being trained for outputting a prediction or estimation of a conductivity map that corresponds to an input B 1 field phase map. The input B 1 field map may be an input B 1 phase map. By receiving the input B 1 phase map, the trained DNN may output multiple conductivity maps by applying it multiple times on the same input B1 field map. This may allow an accurate and precise conductivity map reconstruction e.g. of human brain tissues at clinically available MR field strengths.

In a second example, each of the training B1 field maps comprises a B1 amplitude map, wherein the input B1 field map comprises a B1 amplitude map. In other terms, the training is performed using B 1 amplitude maps in order to generate a trained DNN that can estimate or predict a permittivity map in response to receiving as input a B1 amplitude map. The DNN may for example comprise an input layer configured to receive voxel or pixel values of the B1 amplitude maps. Each B1 amplitude map may for example comprise an image whose pixel values may be input to the DNN for performing the training. The output layer of the DNN may for example be configured to provide output values which are indicative of the permittivity values. The training may result in the DNN being trained for outputting a prediction or estimation of a permittivity map that corresponds to an input B 1 amplitude map. The input B1 field map may be an input B1 amplitude map. By receiving the input B1 amplitude map, the trained DNN may output multiple permittivity maps by applying it multiple times on the same input B1 field map. This may allow an accurate and precise permittivity map reconstruction e.g. of human brain tissues at clinically available MR field strengths.

The first and second examples may enable a phase-only conductivity reconstruction and a magnitude-only permittivity reconstruction. This may particularly be advantageous in the frequency range corresponding to MR field strengths below 3 T, where the wave behavior of B1 is less significant, and the absolute B1 phase depends on the conductivity distribution more than the permittivity while permittivity is more closely related to the magnitude of B1 fields.

In a third example, each of the training B1 field maps comprises a respective B1 field phase map and B1 amplitude map, wherein the input B1 field map comprises a B1 phase map and B1 amplitude map. In other terms, the training is performed using both B 1 amplitude and B1 phase maps in order to generate a trained DNN that can estimate or predict a permittivity map and conductivity map in response to receiving as input a B1 amplitude and B1 phase map. The DNN may for example comprise an input layer configured to receive voxel or pixel values of both the B1 amplitude and B1 phase maps. Each B 1 amplitude map and B1 phase map may for example comprise an image, wherein corresponding pixel values of the two images may be input to the input layer of the DNN for performing the training. The output layer of the DNN may for example be configured to provide output values which are indicative of the permittivity and conductivity values. The training may result in the DNN being trained for outputting a prediction or estimation of a permittivity map and conductivity map that correspond to an input B1 field map. By receiving the input B 1 field map, the trained DNN may output multiple conductivity maps and permittivity maps by applying it multiple times on the same input B1 field map. This may allow an accurate and precise permittivity and conductivity map reconstruction e.g. of human brain tissues at clinically available MR field strengths. In another example, and in order to reduce the complexity, two networks may be trained separately for conductivity and permittivity.

According to one embodiment, the training B1 field maps being measured and/or simulated B1 field maps and the corresponding EP maps being simulated EP maps. The simulation data may be obtained by simulating a realistic coil setup and including realistic head models. This may enable to obtain a high number of unique B1 fields. This may overcome the need of a high amount of MR data for training. The simulated B 1 field maps may or may not comprise artifacts. Not using artifacts may speed up the process of predicting EP maps while still obtaining accurate results of the EP maps (e.g. the accuracy may be controlled by checking the accuracy condition). Using measured B 1 field maps (or simulated B1 field maps with artifacts) may enable a robust deep learning of EPT to artifacts arising from in-vivo image acquisition.

According to one embodiment, using the MC dropout comprises controlling the dropout rate, the number and/or position of dropout layers of the DNN. The MC dropout may for example be used in accordance with the method described in Gal, Yarin, und Zoubin Ghahramani. Dropout as a Bayesian Approximation: Representing Model Uncertainty in Deep Learning. arXiv: 1506.02142 [cs, stat], June 2015. http://arxiv.org/abs/1506.02142*.*

According to one embodiment, the DNN is a U-NET. The U-NET may comprise successive layers, where pooling operations may be replaced by upsampling operators. These layers may increase the resolution of the output of the U-NET.

According to one embodiment, the system is configured to connect to one or more MRI systems and to receive the input B1 map and/or the measured B1 maps from the MRI systems.

According to one embodiment, the system further comprises a MRI system, the MRI system being configured for acquiring image data and to reconstruct B1 maps out of the image data, the input B1 map and/or the measured B1 maps comprise the reconstructed B1 maps.

In another aspect, the invention relates to a method for determining electrical properties, EP, of a target volume in a subject. The method comprises: a) training of a deep neural network, DNN, using a training dataset, the training dataset comprising training B1 field maps and corresponding EP maps, the training comprising using a monte carlo, MC, dropout of the DNN during the training, resulting in a trained DNN configured for generating EP maps from B1 field maps; b) receiving an input B1 field map of the target volume, and repeatedly generate by the trained DNN from the input B1 field map an EP map, resulting in a set of EP maps, wherein the generating comprises using in each repetition the MC dropout during inference of the DNN; c) combining the set of EP maps for determining an EP map and associated uncertainty map of the input B1 field map.

In another aspect, the invention relates to a computer program product comprising machine executable instructions for execution by a processor, wherein execution of the machine executable instructions causes the processor to the methods of any of the preceding embodiments.

It is understood that one or more of the aforementioned embodiments of the invention may be combined as long as the combined embodiments are not mutually exclusive.

### BRIEF DESCRIPTION OF THE DRAWINGS

In the following preferred embodiments of the invention will be described, by way of example only, and with reference to the drawings in which:
Fig. 1 is a schematic diagram of a control system in accordance with the present subject matter,
Fig. 2 is a flowchart of a method for determining electrical properties (EP) of a target volume in an imaged subject,
Fig. 3 depicts a block diagram illustrating a training process of a deep neural network.
Fig. 4 shows a cross-sectional and functional view of an MRI system.

### DETAILED DESCRIPTION OF THE EMBODIMENTS

In the following, like numbered elements in the figures are either similar elements or perform an equivalent function. Elements which have been discussed previously will not necessarily be discussed in later figures if the function is equivalent.

Various structures, systems and devices are schematically depicted in the figures for purposes of explanation only and so as to not obscure the present invention with details that are well known to those skilled in the art. Nevertheless, the attached figures are included to describe and explain illustrative examples of the disclosed subject matter.

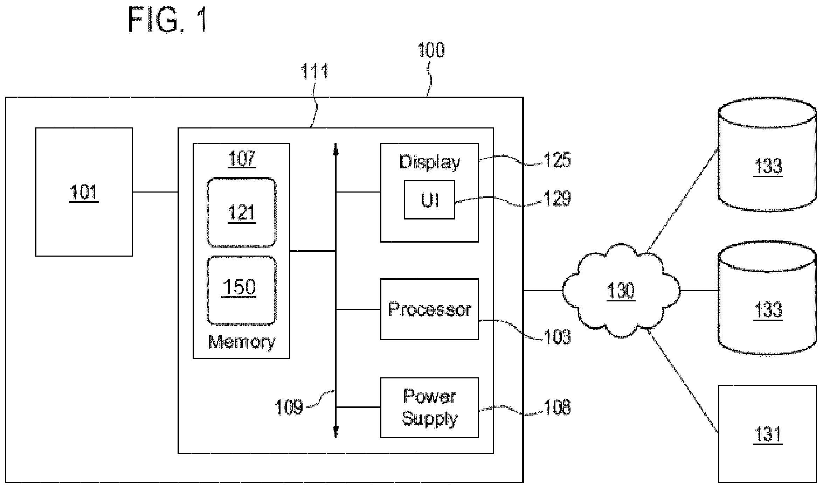

Fig. 1 is a schematic diagram of a medical analysis system 100. The medical analysis system 100 comprises a control system 111 that is configured to connect to a scanning imaging system (or acquisition component) 101. The control system 111 comprises a processor 103, a memory 107 each capable of communicating with one or more components of the medical system 100. For example, components of the control system 111 are coupled to a bidirectional system bus 109.

It will be appreciated that the methods described herein are at least partly non-interactive, and automated by way of computerized systems. For example, these methods can further be implemented in software 121, (including firmware), hardware, or a combination thereof. In exemplary embodiments, the methods described herein are implemented in software, as an executable program, and is executed by a special or general-purpose digital computer, such as a personal computer, workstation, minicomputer, or mainframe computer.

The processor 103 is a hardware device for executing software, particularly that stored in memory 107. The processor 103 can be any custom made or commercially available processor, a central processing unit (CPU), an auxiliary processor among several processors associated with the control system 111, a semiconductor based microprocessor (in the form of a microchip or chip set), a micro-processor, or generally any device for executing software instructions. The processor 103 may control the operation of the scanning imaging system 101.

The memory 107 can include any one or combination of volatile memory elements (e.g., random access memory (RAM, such as DRAM, SRAM, SDRAM, etc.)) and non-volatile memory elements (e.g., ROM, erasable programmable read only memory (EPROM), electronically erasable programmable read only memory (EEPROM), programmable read only memory (PROM). Note that the memory 107 can have a distributed architecture, where various components are situated remote from one another, but can be accessed by the processor 103. Memory 107 may store an instruction or data related to at least one other constituent element of the medical analysis system 100.

The control system 111 may further comprise a display device 125 which displays characters and images and the like e.g. on a user interface 129. The display device 125 may be a touch screen display device.

The medical analysis system 100 may further comprise a power supply 108 for powering the medical analysis system 100. The power supply 108 may for example be a battery or an external source of power, such as electricity supplied by a standard AC outlet.

The scanning imaging system 101 may comprise at least one of MRI, CT and PET-CT imagers. The control system 111 and the scanning imaging system 101 may or may not be an integral part. In other terms, the control system 111 may or may not be external to the scanning imaging system 101.

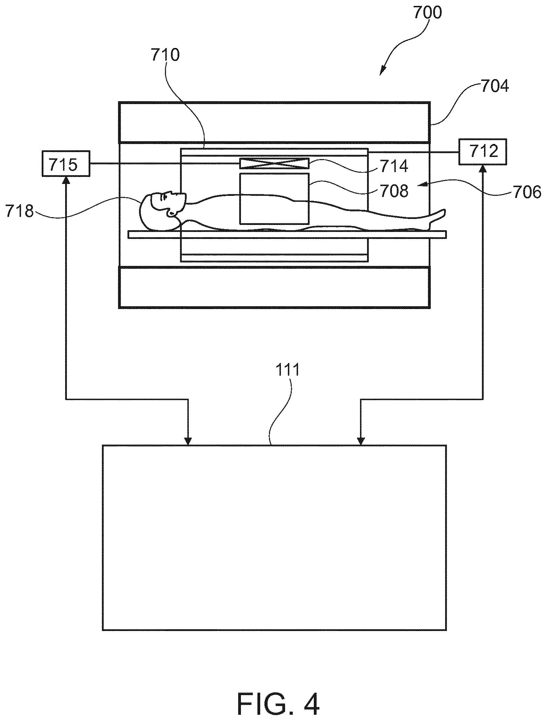

The scanning imaging system 101 comprises components that may be controlled by the processor 103 in order to configure the scanning imaging system 101 to provide image data to the control system 111. The configuration of the scanning imaging system 101 may enable the operation of the scanning imaging system 101. The operation of the scanning imaging system 101 may for example be automatic. Fig. 4 shows an example of components of the scanning imaging system 101 being an MRI system.

The connection between the control system 111 and the scanning imaging system 101 may for example comprise a BUS Ethernet connection, WAN connection, or Internet connection etc.

In one example, the scanning imaging system 101 may be configured to provide output data such as images in response to a specified measurement. The control system 111 may be configured to receive data such as MR image data from the scanning imaging system 101. For example, the processor 103 may be adapted to receive information (automatically or upon request) from the scanning imaging system 101 in a compatible digital form so that such information may be displayed on the display device 125. Such information may include operating parameters, alert notifications, and other information related to the use, operation and function of the scanning imaging system 101.

The medical analysis system 100 may be configured to communicate via a network 130 with other scanning imaging systems 131 and/or databases 133. The network 130 comprises for example a wireless local area network (WLAN) connection, WAN (Wide Area Network) connection LAN (Local Area Network) connection or a combination thereof. The databases 133 may comprise information relates to patients, scanning imaging systems, anatomies, scan geometries, scan parameters, scans etc. The databases 133 may for example comprise an electronic medical record (EMR) database comprising patients' EMR, Radiology Information System database, medical image database, PACS, Hospital Information System database and/or other databases comparing data that can be used for planning a scan geometry. The databases 133 may for example comprise training datasets for the training performed by the present subject matter.

The memory 107 may further comprise an artificial intelligence (AI) component 150. The AI component 150 may or may not be part of software component 121. The AI component 150 may be configured for training a DNN in accordance with the present subject matter and to provide the trained DNN for further use. For example, if the control system 111 is not part of the scanning imaging system 101, the trained DNN may be provided to the scanning imaging system 101 such that it can be used at the scanning imaging system 101 for determining EP maps.

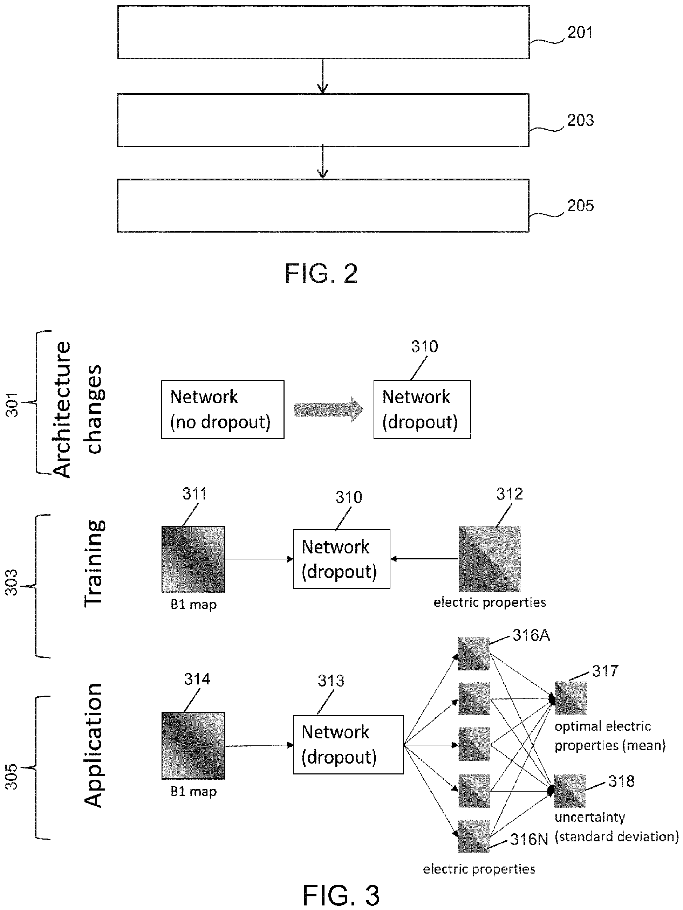

Fig. 2 is a flowchart of a method for determining electrical properties (EP) of a target volume of a subject. The subject may be imaged using an MRI system to acquire MRI data that can be used to perform an electrical properties tomography (EPT). The EPT method may be used to map the dielectric properties (electrical conductivity and permittivity) of tissue at the Larmor frequency. The electric properties of the portions of the subject of interest can potentially be used as additional information for supporting diagnostics with the object to discriminate healthy tissue from malign tissue, e.g. a tumor.

In step 201, a DNN may be trained using a training dataset. The training dataset comprises training B1 field maps and corresponding EP maps. The training comprises using a MC dropout of the DNN during the training. This results in a trained DNN configured for generating EP maps from B1 field maps.

For example, the DNN with MC dropout can intuitively be understood as an ensemble of a large number of similar but different subnetworks with partially shared weights. Network application corresponds to randomly sampling one of the subnetworks from this ensemble. Therefore, a high similarity of output values for repeated network evaluations may correspond to high agreement between different subnetworks.

In one example, the training may be performed using simulation data. The training dataset comprises simulated B1 field maps and associated simulated EP maps (the simulated EP maps may be labels of the simulated B1 field maps). The simulated EP maps may be obtained by EP models and may thus provide realistic labels. Using simulation data may be advantageous as it may increase accuracy and variability of training data. The accuracy relates to the degree in which B1 map and EPs fulfil the physical relationship determined by the Helmholtz equation. For example, by inheriting a potential ground truth's corruption, prediction accuracy of the trained model reaches the average accuracy of the ground truth at maximum. The variability refers to geometrical variability. Experiments show that neural networks are able to approximate the desired underlying functionality to great accuracy for prediction data that is similar to the training data. Large variability within the training set has a beneficial effect by increasing the likelihood of similarity between prediction data and training data. The simulated B1 field maps may be provided with or without artifacts. This may be advantageous as a simulation of artifacts may not be accurate or reliable simulation. Due to the huge variety of possible in-vivo artifacts, a simulation of these artifacts may be too cumbersome.

In another example, the training may be performed using measured B1 field maps and simulated EP maps. The simulated EP maps may be labels of the measured B1 field maps. This may enable to take into account a realistic artifact in the B1 field maps and thus may reduce the difference between the training dataset and the test dataset using in the inference stage.

In step 203, the trained DNN may repeatedly generate from a received input B1 field map an EP map. This results in a set of EP maps, wherein the generating comprises using in each repetition the MC dropout during inference or usage or application of the trained DNN on the input B 1 field map. The number of repetitions may for example be a predefined number e.g. 10 times. The optimal number of repetitions may be a trade-off between accuracy and reconstruction time e.g. the number of repetitions may be such that the accuracy of the EP maps is better than a predefined accuracy threshold and that the reconstruction time is smaller than a predefined maximum reconstruction time.

In step 205, the set of EP maps maybe combined for determining an EP map and associated uncertainty map of the input B 1 field map. The combination may for example comprise averaging the set of EP maps e.g. the corresponding pixel values may be averaged to obtain an averaged EP map. The present method may be advantageous because using the uncertainty map may enable to detect faulty network behavior and thus control the usage of the DNNs in estimating EP maps.

Fig. 3 depicts a block diagram illustrating a training process of a deep neural network 310. Figure 3 illustrates three processing stages, namely a pre-processing stage 301, a training stage 303 and an application stage 305.

In the pre-processing stage 301, an architecture of a DNN may be changed by introducing dropout layers in the DNN. At this stage various degrees of freedom may be tuned specifically for each training dataset, including the number and position of dropout layers and the dropout rate. Dropout rate and number of dropout layers relate to the differences between individual subnetworks and their complexity with respect to the full network. These choices may therefore correspond to nonlinear scaling of the resulting uncertainty value.

In the training stage 303, the DNN with dropout may be trained with known B1-EP-pairs. The DNN 310 may be trained using a predefined training dataset. The training dataset comprise B1 field maps 311 and corresponding labels which are EP maps 312. The training of the DNN 310 may be performed with MC dropout. This results in a trained DNN 313.

In the application stage 305, the trained DNN 313 may be used to repeatedly generate from an input B1 field map 314 a set of EP maps 316A-N (e.g. 5 EP maps). The set of EP maps 316A-N may be combined to compute the mean EP map 317 and to compute the uncertainty map 318 having standard deviation values. In other words, the network 313 is evaluated repeatedly for each input. Mean of output values is used as optimal estimation and standard deviation as uncertainty. In the application stage 305, the MC dropout remains turned on during network application, yielding different EPs for repeated evaluation of the same input. Optimal EPs and uncertainty are calculated by taking mean and standard deviation of the intermediate output EPs 316A-N.

Fig. 4. illustrates a magnetic resonance imaging system 700 as an example of the medical system 100. The magnetic resonance imaging system 700 comprises a magnet 704. The magnet 704 is a superconducting cylindrical type magnet with a bore 706 in it. The use of different types of magnets is also possible; for instance, it is also possible to use both a split cylindrical magnet and a so called open magnet or a sealed magnet. A split cylindrical magnet is similar to a standard cylindrical magnet, except that the cryostat has been split into two sections to allow access to the iso-plane of the magnet. Such magnets may for instance be used in conjunction with charged particle beam therapy. An open magnet has two magnet sections, one above the other with a space in-between that is large enough to receive a subject 718 to be imaged, the arrangement of the two sections area similar to that of a Helmholtz coil. Inside the cryostat of the cylindrical magnet there is a collection of superconducting coils. Within the bore 706 of the cylindrical magnet 704 there is an imaging zone or volume or anatomy 708 where the magnetic field is strong and uniform enough to perform magnetic resonance imaging.

Within the bore 706 of the magnet there is also a set of magnetic field gradient coils 710 which is used during acquisition of magnetic resonance data to spatially encode magnetic spins of a target volume within the imaging volume or examination volume 708 of the magnet 704. The magnetic field gradient coils 710 are connected to a magnetic field gradient coil power supply 712. The magnetic field gradient coils 710 are intended to be representative. Typically, magnetic field gradient coils 710 contain three separate sets of coils for the encoding in three orthogonal spatial directions. A magnetic field gradient power supply supplies current to the magnetic field gradient coils. The current supplied to the magnetic field gradient coils 710 is controlled as a function of time and may be ramped or pulsed.

MRI system 700 further comprises an RF coil 714 at the subject 718 and adjacent to the examination volume 708 for generating RF excitation pulses. The RF coil 714 may include for example a set of surface coils or other specialized RF coils. The RF coil 714 may be used alternately for transmission of RF pulses as well as for reception of magnetic resonance signals e.g., the RF coil 714 may be implemented as a transmit array coil comprising a plurality of RF transmit coils. The RF coil 714 is connected to one or more RF amplifiers 715.

The magnetic field gradient coil power supply 712 and the RF amplifier 715 are connected to a hardware interface of control system 111. The memory 107 of control system 111 may for example comprise a control module. The control module contains computer-executable code which enables the processor 103 to control the operation and function of the magnetic resonance imaging system 700. It also enables the basic operations of the magnetic resonance imaging system 700 such as the acquisition of magnetic resonance data.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a 'circuit', 'module' or 'system'. Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, a data may be retrieved over a modem, over the internet, or over a local area network. Computer executable code embodied on a computer readable medium may be transmitted using any appropriate medium, including but not limited to wireless, wireline, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

A 'computer memory' or 'memory' is an example of a computer-readable storage medium. A computer memory is any memory which is directly accessible to a processor. A 'computer storage' or 'storage' is a further example of a computer-readable storage medium. A computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising 'a processor' should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the 'C' programming language or similar programming languages and compiled into machine executable instructions. In some instances, the computer executable code may be in the form of a high-level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It will be understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device'. A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, gear sticks, steering wheel, pedals, wired glove, dance pad, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bistable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word 'comprising' does not exclude other elements or steps, and the indefinite article 'a' or 'an' does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: medical system
- 101: scanning imaging system
- 103: processor
- 107: memory
- 108: power supply
- 109: bus
- 111: control system
- 121: software
- 125: display
- 129: user interface
- 133: databases
- 201-205: method steps
- 301: pre-processing stage
- 303: training stage
- 305: application stage
- 310: DNN
- 311: B1 map
- 312: EP map
- 313: trained DNN
- 314: B1 map
- 316A-N: EP maps
- 317: EP map
- 318: uncertainty map
- 700: magnetic resonance imaging system
- 704: magnet
- 706: bore of magnet
- 708: imaging zone
- 710: magnetic field gradient coils
- 712: magnetic field gradient coil power supply
- 714: radio-frequency coil
- 715: RF amplifier
- 718: subject

## Claims

1. A medical analysis system (100) for determining electrical properties, EP, of a target volume (708) in a subject (718), the medical analysis system comprising at least one processor (103); and at least one memory (107) storing machine executable instructions, the processor (103) being configured for controlling the medical analysis system (100), wherein execution of the machine executable instructions causes the processor (107) to:
- train (201) a deep neural network, DNN, using a training dataset, the training dataset comprising training B1 field maps and corresponding EP maps, the training comprising using a monte carlo, MC, dropout of the DNN during the training, resulting in a trained DNN configured for generating EP maps from B1 field maps;
- receive (203) an input B1 field map of the target volume, and repeatedly generate by the trained DNN from the input B1 field map an EP map, resulting in a set of EP maps, wherein the generating comprises using in each repetition the MC dropout during inference of the DNN;
- combine (205) the set of EP maps for determining an EP map and associated uncertainty map of the input B1 field map.

2. The system of claim 1, wherein execution of the machine executable instructions further causes the processor to determine if the uncertainty map fulfils a predefined quality condition, and in response to determining that the uncertainty map does not fulfil the predefined quality condition, re-train the DNN using a further training dataset, and repeat steps b)-c) using the retrained DNN instead of the trained DNN.

3. The system of claim 2, the further training dataset is larger than the training dataset.

4. The system of claim 2, wherein execution of the machine executable instructions further causes the processor to: perform the repeating until the uncertainty map fulfils the predefined quality condition.

5. The system of any of the preceding claims, the training B1 field maps comprise B1 field phase maps and B1 amplitude maps, wherein the input B1 field map comprises a B1 phase map and/or B1 amplitude map.

6. The system of any of the preceding claims, the training B1 field maps being measured and/or simulated B1 field maps and the corresponding EP maps being simulated EP maps.

7. The system of any of the preceding claims, wherein using the MC dropout comprises controlling the dropout rate, the number and/or position of dropout layers.

8. The system of any of the preceding claims, the DNN being a U-NET.

9. The system of any of the preceding claims, being configured to connect to one or more MRI systems and to receive the input B1 field map and/or the measured B1 maps from the MRI systems.

10. The system of any of the preceding claims, further comprising a MRI system, the MRI system being configured for acquiring image data and to reconstruct B1 maps out of the image data, the input B1 map and/or the measured B1 maps comprise the reconstructed B1 maps.

11. A method for determining electrical properties, EP, of a target volume (708) in a subject (718), the method comprising:
- training (201) a deep neural network, DNN, using a training dataset, the training dataset comprising training B1 field maps and corresponding EP maps, the training comprising using a monte carlo, MC, dropout of the DNN during the training, resulting in a trained DNN configured for generating EP maps from B1 field maps;
- receiving (203) an input B1 field map of the target volume, and repeatedly generate by the trained DNN from the input B1 field map an EP map, resulting in a set of EP maps, wherein the generating comprises using in each repetition the MC dropout during inference of the DNN;
- combining (205) the set of EP maps for determining an EP map and associated uncertainty map of the input B1 field map.

12. A computer program product comprising machine executable instructions for execution by a processor, wherein execution of the machine executable instructions causes the processor to perform at least part of the method of claim 11.
